# EUROPEAN PATENT APPLICATION

(11) **EP 4 644 896 A1**
(43) Date of publication of application: **05.11.2025**
(21) Application number: 24174078.6
(22) Date of filing: 03.05.2024
(51) Int. Cl.: G01N 33/15, G01N 21/31, G01N 21/3563, G01N 21/95, A61J 3/10

(54) **CALIBRATION DEVICE AND METHOD FOR CALIBRATING A TABLET SENSOR FOR A TABLET PRESS**

(71) Applicant: Fette Compacting GmbH, 21493 Schwarzenbek (DE)
(72) Inventor: Lüdemann, Stefan, 21035 Hamburg (DE); Kolbe, Sven, 21514 Büchen (DE); Krumme, Markus, 4123 Allschwil (CH); Pellegatti, Laurent, 64880 Habsheim (FR); Roggo, Yves, 68510 Sierentz (FR)
(74) Representative: Hauck Patent- und Rechtsanwälte PartmbB

(57) **Abstract**

The invention relates to a calibration device wherein the calibration device comprises a tablet sensor configured to measure a content property of tablets, wherein the calibration device further comprises a calibration disk and a rotary drive for rotating the calibration disk about a rotational axis, wherein the calibration disk comprises a plurality of tablet receptacles, said tablet receptacles each configured to accommodate a pre-produced tablet with a known content property in a fixed position during rotation of the calibration disk, wherein the tablet sensor is arranged above the calibration disk such that tablets accommodated in the tablet receptacles rotate together with the calibration disk through a measuring field of the tablet sensor, wherein the tablet sensor obtains measuring data of a content property of the tablets in the measuring field, and wherein the calibration device further comprises a control device, said control device receiving the measuring data from the tablet sensor, and being configured to calibrate the tablet sensor on basis of the received measuring data and the known property contents of the tablets. The invention also relates to a corresponding method.

## Description

The invention relates to a calibration device and method for calibrating a tablet sensor for a tablet press, said tablet sensor being configured to measure a content property of tablets.

A tablet sensor for measuring a content property of tablets produced in a tablet press, in particular a content of an active pharmaceutical ingredient (API) is known for example from EP 1 568 480 B1. Apart from one or more APIs, tablets produced in tablet presses usually further comprise one or more excipients. The overall weight of tablets produced in the tablet press is known and can for example be measured. Based on measuring data from the tablet sensor regarding for example the content of an API in the tablet, it can be evaluated whether the API content and weight of tablets produced in the tablet press is within specified parameters.

The tablet sensor disclosed in EP 1 568 480 B1 is a NIR or LIF sensor which is arranged in the compression chamber of the tablet press such that it obtains measuring data of tablets produced after the upper punches of the tablet press have been retracted from the cavities of the die disk. The tablet sensor is stationary disposed above the die plate such that tablets still positioned within a cavity of the die disk pass through the measuring field of the tablet sensor.

There is a need to calibrate tablet sensors for example of the type disclosed in EP 1 568 480 B1, in particular when a new product mixture is to be compressed in a tablet press. More specifically, it is necessary to obtain measuring data with the tablet sensor for tablets produced with the respective product mixture. The calibration can be done on basis of known content properties of the different ingredients of the product mixture. In order to calibrate the tablet sensor arranged within the compression chamber of the tablet press, the tablet press must be placed in regular production operating condition for continuous production with the new product mixture such that tablets produced in the tablet press pass through the measuring field of the tablet sensor for calibration. For tablet presses of the kind concerned, in particular high-capacity rotary tablet presses, a considerable amount of product must be filled into the filling device of the tablet press such that the tablet press can reach regular production conditions. Also, a considerable amount of tablets must be produced during the calibration procedure. The product mixture used as well as the tablets produced are waste after the calibration procedure is finished. Especially in early product development phases of pharmaceutical tablets the product mixture is particularly expensive and difficult to make available. In usual rotary tablet presses the minimum amount of product material for reaching production conditions is several kilograms. Of course, each tablet produced can only be used once for calibration purposes.

In order to reach normal production conditions of the tablet press it is further necessary to adjust all production parameters of the tablet press, in particular for a new product. This relates for example to the adjustment of the filling station or the pressing station as well as the adjustment of rotational speeds of the rotor and for example filling paddles in filling devices. This is a complex and time-consuming task and further increases costs of the calibration process. Furthermore, the measuring data obtained by the tablet sensor during calibration depends on a plurality of adjustment parameters and other boundary conditions. For the calibration, in particular the creation of a measuring model for the product mixture in question, all of these parameters and boundary conditions have to be controlled and documented. Any mistakes made in this regard have considerable effects on the calibration of the tablet sensor.

In order to avoid at least some of the above explained disadvantages a static measurement of tablets being manually placed in the measuring field of the tablet sensor has been suggested. In this way, the use of a large amount of production material and the other above explained disadvantages associated with calibrating the tablet sensor in continuous production operation of the tablet press can be avoided.

However, the relative movement between a tablet and the tablet sensor in normal production of the tablet press has a considerable influence on the measuring results. During production, a tablet enters the measuring field of the tablet sensor, rotates through the measuring field and exits the measuring field again. The tablet sensor thus usually obtains measuring data not only on a specific point of the tablet surface, but rather along a trajectory over the surface of the tablet passing through the measuring field. Any influences of this dynamic measurement cannot be considered during a static calibration process, where the tablet sensor only obtains measuring data from a specific point on the surface of the tablet. Also, the influence of different rotational speeds of the rotor of the tablet press on the obtained measuring data cannot be taken into account. Calibration of the tablet sensor on basis of a static measurement has thus led to considerable deviations with regard to the actual measuring data obtained during a subsequent production process. This makes complex and difficult adjustments necessary during production or the data even unsuited to transfer to a rotary press.

Starting from the above explained prior art it is an object of the present invention to provide a calibration device and method of the above explained type which enables a precise calibration of a tablet sensor for a tablet press in a simple, flexible and cost-efficient manner.

The invention solves this object on basis of the independent claims. Advantageous embodiments are the subject of the dependent claims, as well as the specification and the drawings.

For a calibration device of the type explained above the invention solves the object, in that the calibration device comprises a tablet sensor configured to measure a content property of tablets, that the calibration device further comprises a calibration disk and a rotary drive for rotating the calibration disk about a rotational axis, wherein the calibration disk comprises a plurality of tablet receptacles, said tablet receptacles each configured to accommodate a pre-produced tablet with a known content property in a fixed position during rotation of the calibration disk, wherein the tablet sensor is arranged above the calibration disk such that tablets accommodated in the tablet receptacles rotate together with the calibration disk through a measuring field of the tablet sensor, wherein the tablet sensor obtains measuring data of a content property of the tablets in the measuring field, and that the calibration device further comprises a control device, said control device receiving the measuring data from the tablet sensor, and being configured to calibrate the tablet sensor on basis of the received measuring data and the known property contents of the tablets.

For a method of the above explained type the invention solves the object by the steps:
- pre-produced tablets with a known content property are accommodated in a plurality of tablet receptacles of a calibration disk,
- the calibration disk is rotated about a rotational axis, wherein the tablets are in a fixed position in the tablet receptacles during rotation of the calibration disk,
- the tablet sensor is arranged above the calibration disk such that tablets accommodated in the tablet receptacles rotate together with the calibration disk through a measuring field of the tablet sensor,
- the tablet sensor obtains measuring data of a content property of the tablets in the measuring field,
- the tablet sensor is calibrated on basis of the measuring data and the known property contents of the tablets.

The inventive calibration device is not a (rotary) tablet press, but is arranged separately from a tablet press in which the tablet sensor to be calibrated shall be used during production. Such a tablet press may be a rotary tablet press. Consequently, the inventive calibration device in particular does not comprise a filling station or a pressing station or an ejector station as provided by a tablet press. Preferably, the calibration disk only comprises the tablet receptacles. The tablet receptacles are arranged such that tablets are held in a fixed position in the tablet receptacles during rotation, in particular during at least one full rotation, preferably multiple full rotations, of the calibration disk. In particular, tablets cannot fall through the receptacles during rotation of the calibration disk.

The tablet receptacles are adapted to the form of the tablets to be measured, in particular have a form complementary to the form of the tablets. The tablet receptacles are in particular formed as recesses or depressions in the calibration disk. The tablet receptacles are in particular not bores with an open bottom. Rather, the tablet receptacles are closed at the bottom such that tablets accommodated in the receptacles cannot exit the receptacles through the bottom. The calibration disk may for example comprise more than five receptacles, preferably at least 10 receptacles, more preferably more than 10 receptacles. The tablet receptacles are arranged along a circle on the calibration disk, preferably at regular intervals. The receptacles preferably have the same form and orientation on the calibration disk.

A rotary drive is provided which drives the calibration disk to rotate about a rotational axis, which may for example be a vertical rotational axis. During rotation, tablets remain in a fixed position in the tablet receptacles, as explained above. In particular, tablets are not moved in an axial direction parallel to the rotational axis of the calibration disk during rotation of the calibration disk. Due to this embodiment of the calibration disk, the exact position of the tablets to be measured is reliably defined such that the measuring trajectory of the tablet sensor on the surface of the tablets is the same for each tablet.

The tablets arranged in the tablet receptacles of the calibration disk are pre-produced, i.e. they are not produced in the calibration device. The pre-produced tablets used during the inventive calibration process may for example have been produced in a small batch press, such as an experimental press, or even in a press with only a single pair of an upper and lower punch.

The invention is based on the idea to provide a calibration device which can simulate the conditions within a tablet press precisely without the above explained disadvantages of calibrating the tablet sensor within the compression chamber of the tablet press. The rotating calibration disk with the tablets contained in the tablet receptacles simulates the rotating die disk of a tablet press, and thus the relative movement between the tablets to be measured and the tablet sensor as is present in the tablet press. Influences of further specific parameters and boundary conditions of the tablet press, which have to be controlled and documented in a complex matter as explained above, can be largely avoided. By providing a plurality of tablet receptacles in the calibration disk it is possible to obtain measuring data for a plurality of pre-produced tablets during rotation of the calibration disk. The obtaining of measuring data for several tablets is necessary for statistical methods used during the formation of a model for the specific product mixture used for pressing the tablets. For example, tablets with different known contents of a specific ingredient, for example an active pharmaceutical ingredient, can be placed in the tablet receptacles such that a content curve for different ingredient contents can be obtained based on the measuring data from the tablet sensor. Such content curves can be used during production in the actual tablet press for determining the specific ingredient content measured by the tablet sensor. As explained above, the overall weight of the tablets is usually known or can be measured in the production process of the tablet press. Based on the measurement values on the content of certain ingredients, also the weight of these ingredients in a tablet can be reliably determined on this basis.

The inventive calibration device and method also allow repeatedly measuring the same tablets during a calibration process, and thus further minimizing the product amount necessary for the calibration. The essential parameters influencing the measurement results, for example the distance between the tablet sensor and the tablet, the rotational speed of the tablet during measurement, as well as the measurement points on the surface of the tablet during rotation, can be reliably created in the calibration device.

The invention thus allows the calibration and the creation of a corresponding measuring model with a minimum amount of product. In extreme cases, a single tablet could be sufficient for the creation of a first model. In this way, the model creation for new products can be achieved considerably earlier in the development process. Obtaining early data accelerates reaching process safety for the production process and time to market as an essential factor for maximizing gains, for example since after expiry of patents, possible selling prices reduce considerably. The use of smaller amounts of product reduces development costs considerably, bearing in mind sometimes extremely expensive products. By reducing parameters influencing the measurement data to a minimum, the quality of the model and the calibration can be significantly increased. For example, influencing factors, such as a tolerance of the length and dimensions of press punches, the adjustment of an ejector, the expansion of a press frame or deviations in the filling system are completely avoided.

The calibration device is reduced to a necessary minimum and thus much cheaper than an actual tablet press. Calibration can be carried out in a shorter timeframe and is less complex such that costs are reduced. Furthermore, since for the inventive calibration process pre-produced tablets are used, dust creation can be avoided, which may be a hazard to operating personnel. The calibration device thus poses less demands regarding health and safety and can be used more flexibly. Also, as will be explained in more detail below, the invention allows calibration and model finding for a large variety of different types of tablet presses.

According to an embodiment the rotary drive may comprise an electric motor, in particular a servo motor. Such electric motors can be flexibly and individually controlled to enable simulating different rotor speeds of a tablet press. The control device may be configured to control the rotary drive to rotate the calibration disk at different rotational speeds for different calibration processes.

The content property may be a content of an active pharmaceutical ingredient (API) in the tablet. As explained above, tablets usually comprise one or more APIs and one or more excipients. Of course, it is necessary to precisely determine in particular the content of an API in a tablet.

The tablet sensor may be a spectroscopic sensor, preferably a NIR or LIF or Raman sensor. Such a sensor is particularly suited to measure for example an API content. Such sensors comprise an emitter emitting electromagnetic radiation, for example in the near infrared wavelength range, onto the surface of the tablet and a receiver receiving electromagnetic radiation reflected back by the surface of the tablet. The radiation may be focused on the surface of the tablet. By evaluating the spectrum of the reflected radiation, it is possible to determine the content of certain ingredients in the tablet.

The tablet sensor may be positioned on a calibration table, wherein the calibration table also accommodates the calibration disk. Also, the rotary drive may be arranged on or under the calibration table, for example held on the underside of the calibration table. The calibration table may be positioned in the same room as a tablet press to be equipped with the tablet sensor or separate from such a production room.

According to a further embodiment the tablet sensor may have attachment means configured to cooperate with attachment means of the calibration table, wherein the tablet sensor can be removably attached to the calibration table through cooperation of the attachment means of the calibration table with the attachment means of the tablet sensor. This allows for the tablet sensor to be easily installed in and removed from the calibration device. In particular, it allows to easily swap the tablet sensor between the calibration device and the tablet press.

The tablet sensor of the calibration device is preferably the same tablet sensor to be installed in the tablet press. This of course avoids potential influences of specific characteristics of a specific tablet sensor on the measuring data. According to a further embodiment the attachment means of the tablet sensor may be configured to cooperate with attachment means in a compression chamber of a tablet press such that the tablet sensor can also be removably attached in the compression chamber of the tablet press. In this manner the same tablet sensor to be used in the tablet press can be easily installed in the calibration device for calibration, and vice versa. Generally, the tablet sensor may comprise the same components as in the tablet press, for example components to hold and/or adjust the position of the tablet sensor. The inventive calibration device may thus be suited to be equipped with the full tablet sensor arrangement as is provided in the tablet press.

According to a further embodiment it is possible, that the calibration device comprises a plurality of different calibration disks, wherein the calibration disks have different diameters and/or wherein the calibration disks have different amounts and/or forms of tablet receptacles and/or tablet receptacles arranged on circles with different diameters, and in that the calibration disks are each provided with attachment means configured to cooperate with attachment means of the calibration table, wherein the calibration disks can alternatively be removably attached to the calibration table through cooperation of the attachment means of the calibration table with the attachment means of the calibration disks. This embodiment allows for flexible adaptation of the calibration device to simulate different types and configurations of tablet presses and their components, such as the rotor, as well as the tablets to be produced.

According to a further embodiment the control device may be configured to control the tablet sensor to start a measuring procedure when a tablet to be measured enters the measuring field of the tablet sensor and to terminate the measuring procedure when the tablet to be measured exits the measuring field of the tablet sensor. According to a further embodiment in this regard the calibration device may further comprise a rotational angle sensor, said rotational angle sensor providing the rotational angle of the calibration disk during rotation to the control device, wherein the control device is configured to control the tablet sensor to start and terminate the measuring procedure based on the rotational angle provided by the rotational angle sensor. The rotational angle sensor can be provided for example by an electric motor of the rotary drive which is able to provide the rotational angle of the calibration disk in certain defined steps. The rotational angle sensor may also be a separate sensor, arranged on the calibration disk or relative to the calibration disk and providing the rotational angle position of the calibration disk during rotation. The rotational angle sensor may for example comprise an angle encoder. According to the above embodiments the control device triggers the tablet sensor to start a measuring procedure when a tablet to be measured enters the measuring field, and to terminate measuring procedure when the tablet to be measured exits the measuring field. Based on these trigger events the tablet sensor then obtains measuring data along a measuring path or trajectory over the surface of the tablet corresponding to the rotation of the tablet together with the calibration disk through the measuring field. In this manner measuring data can be provided over the full extension of the tablet as it travels through the measuring field. The rotational angle sensor may be provided such that angle deviations towards the actual angle position of the calibration disk may be less than 0.8°, preferably less than 0.4°, more preferably less than 0.2°.

According to a further embodiment the calibration device may comprise position adjustment means, said position adjustment means being configured to adjust the position of the tablet sensor relative to the calibration disk in at least one spatial direction, preferably in at least two spatial directions, and more preferably in all three spatial directions. Being able to adjust the tablet sensor in this manner allows to reliably set a focus point of the tablet sensor relative to the surface of the tablet, for example in the vertical or z-direction. It also allows adjusting the position of the tablet sensor relative to different forms of tablets and/or different calibration disks having differently formed or arranged tablet receptacles. According to a further embodiment the control device may be configured to control the position adjustment means. This allows for an automatic adjustment of the position of the tablet sensor.

The calibration device may further comprise an optical reference surface, preferably a white reference surface, wherein the tablet sensor can be positioned such that the optical reference surface is positioned in the measuring field of the tablet sensor, and wherein the control device is configured to balance, in particular to white balance, the tablet sensor against the optical reference surface. The positioning of the reference surface in the measuring field may for example be achieved by adjusting the position of the tablet sensor with position adjustment means. This embodiment improves measuring data.

According to a further embodiment it is possible that the tablet receptacles are formed in receptacle holders, said receptacle holders being releasably fastened in the calibration disk, wherein the calibration device preferably comprises different sets of receptacle holders, which can be alternatively releasably fastened in the calibration disk, wherein the different sets of receptacle holders have differently formed tablet receptacles. In this manner the calibration device, in particular the calibration disk, can be easily adapted to different tablet forms. More specifically, if a different type of tablet shall be used for the calibration process the currently installed set of receptacle holders can be removed and replaced with a different set of receptacle holders having differently formed tablet receptacles. This allows using different types of tablets to be utilized in the calibration process without having to replace the calibration disk.

The invention also pertains to a system comprising a tablet press, in particular a rotary tablet press, and a calibration device according to the present invention.

According to a further embodiment it is possible that the tablet receptacles of the calibration disk are arranged along a circle with a first diameter, and in that cavities in a die disk of a rotor of the tablet press, in which cavities powder material is compressed to tablets by punches of the tablet press during production, are arranged along a circle with a second diameter, wherein the first diameter is smaller than the second diameter. More specifically, according to the above embodiment the calibration disk along with the circle of the tablet receptacles may be provided with a smaller diameter than the die disk of the rotor in the actual tablet press. It is nevertheless possible to achieve the same rotational speed of the calibration disk as a potentially larger die disk in a rotor. More specifically, since in the inventive calibration device and system no powder is being processed, but rather pre-produced tablets are used, losing powder from cavities due for example to centrifugal force, is not an issue. Using a smaller diameter calibration disk and circle of tablet receptacles also reduces height deviations of the surface of a calibration disk during rotation compared to larger diameter disks. Such height deviations are particularly critical for the measuring results. According to the invention height deviations of the upper surface of the calibration disk during a full rotation may be less than 1 mm, preferably less than 0.7 mm, more preferably less than 0.4 mm, and even more preferably less than 0.2 mm.

According to an embodiment of the inventive method it is possible that the tablets accommodated in the tablet receptacles each have the same content property, and in that the calibration disk is rotated by 360° for calibrating the tablet sensor or in that at least some of the tablets accommodated in the tablet receptacles, preferably all of the tablets accommodated in the tablet receptacles, have different content properties, and in that the calibration disk is rotated by 360° several times for calibrating the tablet sensor, preferably at least as often as there are tablets in the tablet receptacles with different content properties. As explained above it is possible to measure one tablet several times in the inventive calibration device and method by rotating the calibration disk by more than 360°, in particular several times for 360°. This increases statistical reliability of the obtained data. It is also possible to rotate the calibration disk only once for example by 360°. Depending on whether the tablet receptacles are fitted with tablets having the same content property or having different content properties it is thus possible to obtain sufficient statistical data for the calibration and building of a model.

The inventive method may be carried out with the inventive calibration device or with the inventive system. Correspondingly, the inventive calibration device or the inventive system may be configured to carry out the inventive method.

An embodiment of the invention will be explained in more detail in the following with reference to drawings. It is shown schematically in
- Figure 1: a calibration device according to the invention in a side view,
- Figure 2: the calibration device shown in figure 1 in a perspective view,
- Figure 3: the calibration device shown in figure 1 in a top view, and
- Figure 4: an inventive system in a side view.

In the drawings the same reference numerals denote the same parts, unless specified otherwise.

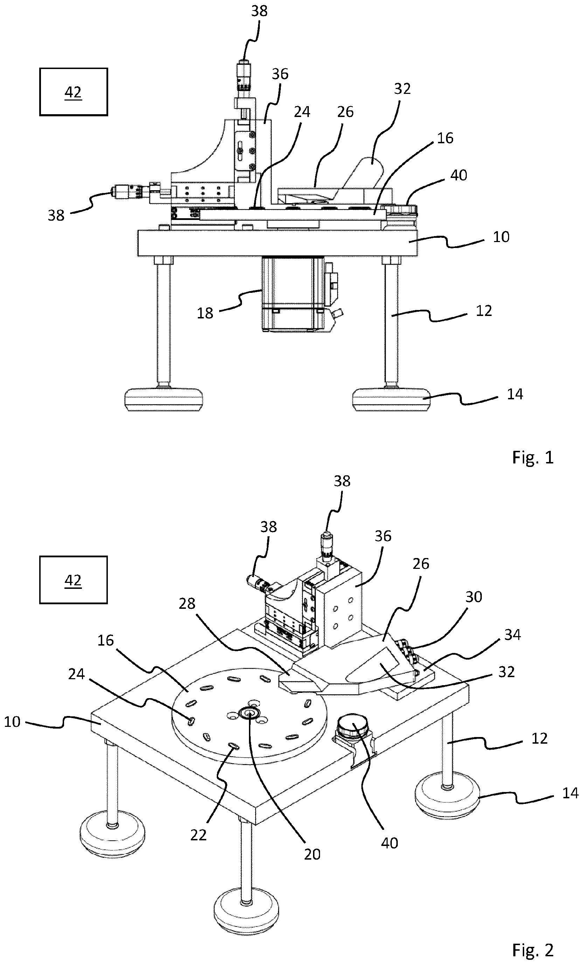

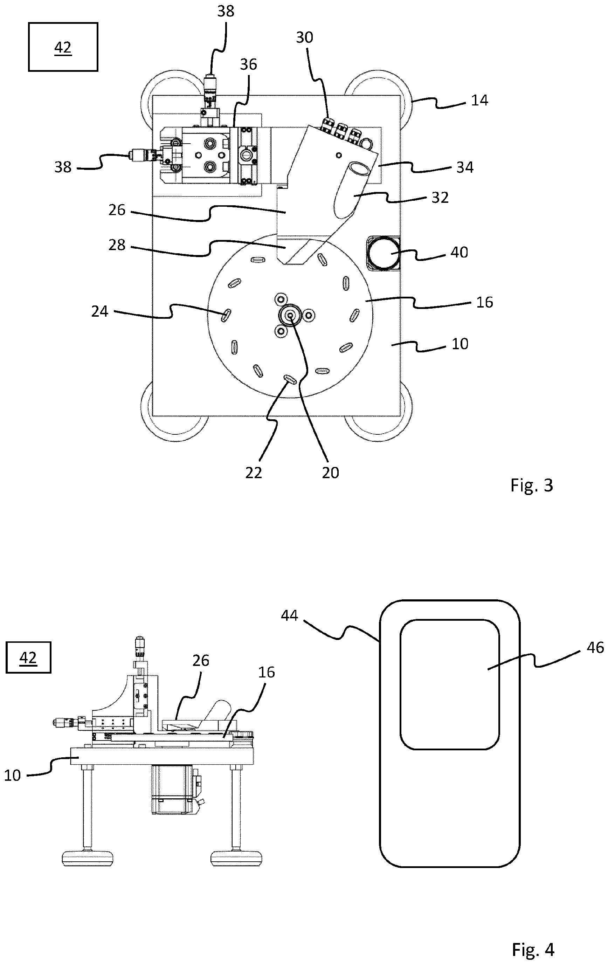

The inventive calibration device shown in Figures 1 to 3 comprises a calibration table 10 with, in the example shown, four legs 12 and feet 14 with which the calibration device can be placed on a floor. On the calibration table 10 a calibration disk 16 is positioned in a rotatable manner. A rotary drive 18, comprising for example an electric motor, such as a servo motor, is arranged underneath the calibration table 10 and can drive the calibration disk 16 to rotate about a rotational axis 20, which in the example shown is a vertical rotational axis 20. The calibration disk 16 comprises a plurality of tablet receptacles 22, each for accommodating a pre-produced tablet 24 in fixed position during rotation of the calibration disk 16. The tablet receptacles 22 each have the same form and are arranged in equal intervals in a circle in the calibration disk 16. As can be seen for example in the top view in figure 3 the tablet receptacles 22, and thus the tablets 24 held in the tablet receptacles 22 are each of oblong shape, wherein a longitudinal direction of the oblong shape is arranged at an angle towards the circle of the tablet receptacles 22. The tablet receptacles 22 are formed as depressions in the calibration disk 16 with a closed bottom and have a complementary form towards the tablets.

The inventive calibration device further comprises a tablet sensor 26, in particular a spectroscopic sensor, like a NIR or LIF or Raman sensor, measuring a content property, such as an API content, of the tablets 24. To this end, a measuring head 28 comprising an emitter and a receiver for electromagnetic radiation is positioned above the calibration disk 16 such that the tablets 24 accommodated in the tablet receptacles 22 rotate together with the calibration disk 16 through a measuring field of tablet sensor 26, in particular of the measuring head 28, and the tablet sensor 26 obtains measuring data of a content property of the tablets 24 in the measuring field. The tablet sensor 26 comprises several connections 30 for connecting for example optical fibers, as well as a suction connection 32 for connection to a suction device, for example a dust suction device for keeping the measuring field free of contaminations.

In the example shown the tablet sensor 26 is arranged on an adjustment plate 34. The tablet sensor 26 further comprises position adjustment means 36 with which the tablet sensor 26 can be moved in all three spatial directions to adjust the position of the tablet sensor 26. In the example shown, adjustment actuators 38 are provided on position adjustment means 36 to adjust the position. The adjustment actuators 38 may be manually actuated or by control device 42 of the calibration device.

The calibration device further comprises an optical reference surface 40, in the example shown a white reference surface 40, also arranged on calibration table 10. With the position adjustment means 36 the tablet sensor 26 can be positioned with the measuring head 28 above the reference surface 40 such that tablet sensor 26 can be balanced against the optical reference surface 40, in particular white balanced.

Control device 42 can control the position adjustment means 36 and may be configured to carry out the balancing of tablet sensor 26 against the reference surface 40. Control device 42 may also be configured to control rotary drive 18 to rotate the calibration disk 16. Measuring data obtained by tablet sensor 26 is provided to control device 42. Control device 42 is also provided with the known content property of the tablets 24 accommodated in the tablet receptacles 22, such that control device 42 can calibrate tablet sensor 26 based on the measuring data received and the known content properties of tablets 24. Control device 42 may trigger tablet sensor 26 to start a measuring procedure when a tablet 24 to be measured enters the measuring field of the tablet sensor 26 and to terminate the measuring procedure when the tablet 24 exits the measuring field of the tablet sensor 26. To this end, the calibration device may be provided with a rotational angle sensor, which provides the rotational angle of the calibration disk 16 during rotation to the control device 42 such that the control device 42 controls tablet sensor 26 to start and terminate the measuring procedure based on the rotational angle provided by the rotational angle sensor. As explained above, the rotational angle sensor may for example be provided by the rotary drive 18.

Tablet sensor 26 is further provided with attachment means which cooperate with attachment means of calibration table 10 such that tablet sensor 26 can be removably attached to calibration table 16 through cooperation of the attachment means. After calibration has been completed, tablet sensor 26 together can thus be removed from calibration table 10 and can be mounted in a tablet press 44, as shown in the system of Figure 4, in particular within the compression chamber 46 of tablet press 44. To this end the attachment means of tablet sensor 26 can cooperate with corresponding attachment means within the compression chamber 46 of the tablet press 44.

### Reference numerals list

- 10: Calibration table
- 12: Legs
- 14: Feet
- 16: Calibration disk
- 18: Rotary disk
- 20: Rotational axis
- 22: Table receptacles
- 24: Tablet
- 26: Tablet sensor
- 28: Measuring head
- 30, 32: Connections
- 34: Adjustment plate
- 36: Adjustment means
- 38: Adjustment actuators
- 40: Reference surface
- 42: Control device
- 44: Tablet press
- 46: Compression chamber

## Claims

1. Calibration device for calibrating a tablet sensor (26) for a tablet press (44), **characterized in that** the calibration device comprises a tablet sensor (26) configured to measure a content property of tablets (24), that the calibration device further comprises a calibration disk (16) and a rotary drive (18) for rotating the calibration disk (16) about a rotational axis (20), wherein the calibration disk (16) comprises a plurality of tablet receptacles (22), said tablet receptacles (22) each configured to accommodate a pre-produced tablet (24) with a known content property in a fixed position during rotation of the calibration disk (16), wherein the tablet sensor (26) is arranged above the calibration disk (16) such that tablets (24) accommodated in the tablet receptacles (22) rotate together with the calibration disk (16) through a measuring field of the tablet sensor (26), wherein the tablet sensor (26) obtains measuring data of a content property of the tablets (24) in the measuring field, and that the calibration device further comprises a control device (42), said control device (42) receiving the measuring data from the tablet sensor (26), and being configured to calibrate the tablet sensor (26) on basis of the received measuring data and the known property contents of the tablets (24).

2. Calibration device according to claim 1, **characterized in that** the rotary drive (18) comprises an electric motor, in particular a servomotor.

3. Calibration device according to one of the preceding claims, **characterized in that** the content property is a content of an active pharmaceutical ingredient in the tablet (24).

4. Calibration device according to one of the preceding claims, **characterized in that** the tablet sensor (26) is a spectroscopic sensor, preferably a NIR or LIF or Raman sensor.

5. Calibration device according to one of the preceding claims, **characterized in that** the tablet sensor (26) is positioned on a calibration table (10), wherein the calibration table (10) also accommodates the calibration disk (16).

6. Calibration device according to claim 5, **characterized in that** the tablet sensor (26) has attachment means configured to cooperate with attachment means of the calibration table (10), wherein the tablet sensor (26) can be removably attached to the calibration table (10) through cooperation of the attachment means (36) of the calibration table (10) with the attachment means of the tablet sensor (26).

7. Calibration device according to claim 6, **characterized in that** the attachment means of the tablet sensor (26) are configured to cooperate with attachment means in a compression chamber (46) of a tablet press (44) such that the tablet sensor (26) can also be removably attached in the compression chamber (46) of the tablet press (44).

8. Calibration device according to one of claims 5 to 7, **characterized in that** the calibration device comprises a plurality of different calibration disks (16), wherein the calibration disks (16) have different diameters and/or wherein the calibration disks (16) have different amounts and/or forms of tablet receptacles (22) and/or tablet receptacles (22) arranged on circles with different diameters, and **in that** the calibration disks (16) are each provided with attachment means (36) configured to cooperate with attachment means of the calibration table (10), wherein the calibration disks (16) can alternatively be removably attached to the calibration table (10) through cooperation of the attachment means of the calibration table (10) with the attachment means of the calibration disks (16).

9. Calibration device according to one of the preceding claims, **characterized in that** the control device (42) is configured to control the tablet sensor (26) to start a measuring procedure when a tablet (24) to be measured enters the measuring field of the tablet sensor (26) and to terminate the measuring procedure when the tablet (24) to be measured exits the measuring field of the tablet sensor (26).

10. Calibration device according to claim 9, **characterized in that** the calibration device further comprises a rotational angle sensor, said rotational angle sensor providing the rotational angle of the calibration disk (16) during rotation to the control device (42), and **in that** the control device (42) is configured to control the tablet sensor (26) to start and terminate the measuring procedure based on the rotational angle provided by the rotational angle sensor.

11. Calibration device according to one of the preceding claims, **characterized in that** the calibration device comprises position adjustment means (36), said position adjustment means (36) being configured to adjust the position of the tablet sensor (26) relative to the calibration disk (16) in at least one spatial direction, preferably in at least two spatial directions, and more preferably in all three spatial directions.

12. Calibration device according to claim 11, **characterized in that** the control device (42) is configured to control the position adjustment means (36).

13. Calibration device according to one of the preceding claims, **characterized in that** the calibration device further comprises an optical reference surface (40), preferably a white reference surface, wherein the tablet sensor (26) can be positioned such that the optical reference surface (40) is positioned in the measuring field of the tablet sensor (26), and **in that** the control device (42) is configured to balance the tablet sensor (26) against the optical reference surface (40).

14. Calibration device according to one of the preceding claims, **characterized in that** the tablet receptacles (22) are formed in receptacle holders, said receptacle holders being releasably fastened in the calibration disk (16), wherein the calibration device preferably comprises different sets of receptacle holders, which can be alternatively releasably fastened in the calibration disk (16), wherein the different sets of receptacle holders have differently formed tablet receptacles (22).

15. System comprising a tablet press (44) and a calibration device according to one of the preceding claims.

16. System according to claim 15, **characterized in that** the tablet receptacles (22) of the calibration disk (16) are arranged along a circle with a first diameter, and **in that** cavities in a die disk of a rotor of the tablet press (44), in which cavities powder material is compressed to tablets (24) by punches of the tablet press (44) during production, are arranged along a circle with a second diameter, wherein the first diameter is smaller than the second diameter.

17. Method for calibrating a tablet sensor (26) for a tablet press (44), said tablet sensor (26) being configured to measure a content property of tablets, **characterized by** the steps:
• pre-produced tablets (24) with a known content property are accommodated in a plurality of tablet receptacles (22) of a calibration disk (16),
• the calibration disk (16) is rotated about a rotational axis (20), wherein the tablets (24) are in a fixed position in the tablet receptacles (22) during rotation of the calibration disk (16),
• the tablet sensor (26) is arranged above the calibration disk (16) such that tablets accommodated in the tablet receptacles (22) rotate together with the calibration disk (16) through a measuring field of the tablet sensor (26),
• the tablet sensor obtains measuring data of a content property of the tablets (24) in the measuring field,
• the tablet sensor is calibrated on basis of the measuring data and the known property contents of the tablets (24).

18. Method according to claim 17, **characterized in that** it is carried out with a calibration device according to one of claims 1 to 14 or with a system according to one of claims 15 or 16.
